# EUROPÄISCHE PATENTANMELDUNG

(11) **EP 0 670 330 A1**
(43) Veröffentlichungstag der Anmeldung: **06.09.1995**
(21) Anmeldenummer: 95102648.3
(22) Anmeldetag: 24.02.1995
(51) Int. Cl.: C07K 5/06, C07K 5/08, C07C 237/04, A61K 38/05, A61K 38/06, A61K 31/16

(54) **Langkettige Alkylamide von Aminosäuren und Peptiden mit antiproliferativen und entzündungshemmenden Eigenschaften**

(30) Priorität: 04.03.1994 DE 4407193
(71) Anmelder: ASTA Medica Aktiengesellschaft, D-01277 Dresden (DE)
(72) Erfinder: Kutscher, Bernhard, Dr., D-63477 Maintal (DE); Bernd, Michael, Dr., D-60136 Frankfurt a. M. (DE); Grossmann, Heinz, Dr., D-93049 Regensburg (DE); Kick, Maria, D-93051 Regensburg (DE); Arp, Jürgen, D-93051 Regensburg (DE); Liefländer, Manfred, Prof. Dr., D-93161 Sinzing (DE); Engel, Jürgen, Prof., D-63755 Alzenau (DE); Voegeli, R., Dr., D-63069 Offenbach (DE)

(57) **Zusammenfassung**

Langkettige unverzweigte Alkylamide der allgemeinen Formel

CH₃-(CH₂)ₙ-NH-R,

wobei n = 11 - 20 und R = Aminosäure, deren D- und L-Enantiomeren, Di- und Tripeptide, durch N-Schutzgruppen derivatisierte Formen und endständige Ethylester, sowie deren physiologisch verträgliche Salzformen als pharmazeutische Wirkstoffe mit antiproliferativen und entzündungshemmenden Eigenschaften.

## Beschreibung

Die Erfindung betrifft neue Lipoaminosäuren, Lipodipeptide und Lipotripeptide, deren Herstellung und Verwendung als pharmazeutische Wirkstoffe.

Die Derivate nach der allgemeinen Formel

CH₃-(CH₂)ₙ-NH-R

wobei n = 11-20 und R= Aminosäure, deren D- und L-Enantiomeren, Di- und Tripeptide, durch N-Schutzgruppen derivatisierte Formen und endständige Ethylester sowie deren physiologisch verträgliche Salzformen als pharmazeutische Wirkstoffe sind geeignet, Tumorwachstum in vitro und in vivo zu beeinflussen und zu unterdrücken, sowie Proteinkinase C zu inhibieren.

Sie sind desweiteren geeignet, in den Arachidonsäurestoffwechsel einzugreifen und durch Phospholipase A₂-Inhibierung bei gewebsentzündlichen Prozessen pharmakologisch wirksam zu sein. Die benannten Derivate sind in der Lage, Lipoxygenase und Cyclooxygenase in niederer mikromokularer (µm) Konzentration zu inhibieren, wobei unspezifische Histaminfreisetzung nicht beobachtet wird. Lipoxygenase, Cyclooxygenase und Phospholipase A₂ erzeugen Abbauprodukte der Arachidonsäurekaskade aus Phospholipiden; solche Arachidonsäuremetaboliten sind inflammatorisch wirksam und maßgeblich an der Pathogenese, bzw. Aufrechterhaltung pathophysiologischer Prozesse wie Asthma bronchiale, Heuschnupfen, Muskelrheumatismus u.a.m. beteiligt.
Aminosäuren und kurzkettige Peptide aus neutralen und/oder basischen Aminosäuren mit langen aliphatischen Resten besitzen häufig amphiphile Eigenschaften. Amphiphile Substanzen zeichnen sich durch gleichzeitige Anwesenheit von hydrophilen und hydrophoben Molekularbereichen aus. Sie zeigen daher eine strukturelle Verwandtschaft mit Membranbausteinen. Lipoaminosäuren mit der Formel H-Orn-NHR.2HBr (R=C₁₆H₃₃, C₁₈H₃₇) und Lipodipeptide mit der Formel H-Orn-Orn-NHC₁₈H₃₇.3HCl und deren Darstellung sind von G. Kellner und M. Liefländer in Z. Naturforsch. B., Chem. Sci. 46(8) 1098 - 1104(1991) beschrieben. Die Verwendung dieser Verbindungen als pharmazeutische Wirkstoffe mit antiproliferativen und entzündungshemmenden Eigenschaften ist jedoch nicht bekannt.
In dem schweizerischen Patent 490 338 (Chem. Abstr. Vol. 73 120 904 b (1970)) ist die Herstellung von Z-L-Lys(BOC)-L-Lys(BOC)-L-Pro-NH-ₙC₁₆H₃₃ angeführt. Eine entsprechende pharmazeutische Verwendung ist jedoch nicht angegeben. In der PCT-Anmeldung WO 8806885 ist unter anderem die Herstellung von H-D-Pro-NH-C₁₆H₃₃ und seine Verwendung als Phosopholipase A₂-Inhibitor beschrieben. Ein Hinweis auf eine antiproliferative Eigenschaft und eine hemmende Wirkung auf das Wachstum von Primär-Tumoren dieser oder verwandter Verbindungen ist jedoch nicht angegeben.
Diese Wirkung hat sich nun in Versuchen mit den Verbindungen der allgemeinen Formel

CH₃-(CH₂)ₙ-NH-R

wobei n = 11 - 20 und R = Aminosäure, deren D- und L-Enantiomeren, Di- und Tripeptide, durch N-Schutzgruppen derivatisierte Formen und endständige Ethylester sowie deren physiologisch verträgliche Salzformen gezeigt.
Der Aminosäurerest R steht insbesondere für Gly, Ala, Val, Leu, Ile, Ser, Thr, Lys, Arg, Asp, Asn, Glu, Gln, Cys, Met, Phe, Tyr, Pro, Trp, His, Orn, Thz. Besonders bevorzugt sind beispielsweise die Aminosäurereste Orn, Lys, Thz. Die Abkürzungen für die einzelnen Aminosäurereste beruhen auf dem Trivialnamen der Aminosäure und sind dem Fachmann bekannt. Die Aminosäuren stammen vorwiegend aus der L-Serie, jedoch sind in gleicher Weise auch Aminosäuren aus der D-Serie wirksam. Die Verknüpfung der Aminosäuren zu den Di- und Tripeptidresten kann bei den basischen Aminosäuren, wie zum Beispiel bei Ornithin und Lysin über die α-Position als auch über die endständige ω-Position vorgenommen werden.
Die langkettige Alkylkette mit (CH₂)ₙ besteht aus n= 11-20, wobei bevorzugt n= 15-19 und insbesondere n= 16-17 ist. Die gesättigte Alkylkette ist unverzweigt. Als Schutzgruppen für die Aminosäuren kommen zum Beispiel in Frage tertiäre Butyloxycarbonylgruppe, Carbobenzoxygruppe beziehungsweise Carbobenzthiogruppe (gegebenenfalls jeweils mit p-Brom oder p-Nitro-benzylrest). Trifluoracetylgruppe, Phthalylrest, o-Nitrophenoxyacetylgruppe, Tritylgruppe, p-Toluolsulfonylgruppe, Benzylgruppe, im Benzolkern substituierte Benzylreste (p-Brom- oder p-Nitrobenzylrest), α-Phenyl-ethylrest. Hierzu wird auch auf das Buch von Jesse P. Greenstein und Milton Winitz, Chemistry of Amino Acids, New York 1961, John Wiley and Sons, Inc., Volume 2, beispielsweise Seite 883 und folgende sowie The Peptides, Volume 2, Ed. E. Gross and J. Meienhofer, Academic Press New York, Tabelle III and IV verwiesen.
Diese Schutzgruppen kommen grundsätzlich auch für den Schutz von weiteren funktionellen Seitengruppen (OH-Gruppen, NH₂-Gruppen) der in Frage kommenden Aminosäuren in Frage.
Vorhandene Hydroxygruppen (Serin, Threonin) werden vorzugsweise durch Benzylgruppen und ähnliche Gruppen geschützt.
Die Verknüpfung der einzelnen Aminosäuren miteinander erfolgt nach den hierfür üblichen Methoden.

Das Überführen der Verbindungen der allgemeinen Formel in ihre Säureadditionssalze kann durch Umsetzen derselben mit Säuren in an sich bekannter Weise durchgeführt werden.

Umgekehrt kann das Freisetzen der Aminosäure-, Di- und Tripeptid-Verbindungen durch Umsetzen ihrer Säureadditionssalze mit Basen durchgeführt werden.
Beispiele für derartige Säureadditionssalze sind Hydrochlorid, Hydrobromid, Sulfat, Phosphat, Fumarat, Glukonat, Tannat, Maleat, Acetat, Citrat, Benzonat, Succinat, Alginat, Pamoat, Malat, Ascorbat, Tartrat und ähnliche. Wenn der Wirkstoff in Tablettenform verabreicht wird, kann die Tablette ein pharmazeutisch annehmbares Verdünnungsmittel, das ein Bindemittel wie zum Beispiel Tragant, Maisstärke oder Gelatine umfaßt, enthalten, sowie ein Zerfallmittel wie Alginsäure, und ein Gleitmittel wie Magnesiumstearat.
Wenn Verabreichung in flüssiger Form erwünscht wird, kann ein Süssmittel und/oder ein Geschmackstoff als Teil des pharmazeutisch annehmbaren Verdünnungsmittels verwendet werden; bei intravenöser Verabreichung wird diese in isotonischer Saline, Phosphatbufferlösungen oder ähnlichem durchgeführt.
Die pharmazeutischen Zusammensetzungen werden herkömmlicherweise den Wirkstoff in Zusammenhang mit einem üblichen, pharmazeutisch annehmbaren Träger enthalten.

Bei Verwendung der Verbindungen der allgemeinen Formel, vorzugsweise in Form von Salzen mit Säuren in der Humanmedizin, kann eine systemische Verabfolgung, entweder durch intravenöse, subkutane oder intramuskuläre Injektion, oder eine sublinguale oder nasale Verabfolgung, in Verbindung mit pharmakologisch verträglichen Trägerstoffen durchgeführt werden.
Es können auch Arzneipräparate für die orale Verabreichung hergestellt werden. Zu diesem Zweck werden die Wirkstoffe mit geeigneten bekannten pharmazeutischen Verdünnungsmitteln vermischt und in an sich bekannter Weise zu Präparaten, wie Tabletten, Kapseln, Suspensionen, Emulsionen, Lösungen oder dispergierbaren Pulvern, verarbeitet.

Feste Zubereitungen können in Kapseln für die orale Verabfolgung gefüllt werden. Derartige Zubereitungen zur Abfüllung in Kapseln können den festen Wirkstoff im Gemisch mit kolloidalem Aluminiumhydroxid, Calciumhydrogenphosphat, oder zusammen mit einem inerten Feststoff wie Lactose enthalten.
Präparate in Form von Tabletten können auf an sich übliche Weise überzogene oder in Form von schäumenden oder nichtschäumenden Präparaten bereitgestellt werden. Hierbei können inerte Verdünnungsmittel oder Trägerstoffe, wie Magnesiumcarbonat oder Lactose, zusammen mit üblichen Sprengmitten, wie Maisstärke und Alginsäure, und Gleitmitteln, wie Magnesiumstearat, verwendet werden.

Zur Verabreichung auf nasalem Wege in Form von Tropfen oder Sprays werden die Verbindungen der allgemeinen Formel vorzugsweise in einem sterilen, wäßrigen Träger eingesetzt, der auch andere gelöste Stoffe, wie Puffer oder Konservierungsmittel, sowie zur Herstelllung einer isotonen Lösung ausreichende Mengen an pharmakologisch verträglichen Salzen oder an Glucose enthalten kann.
Die Peptide der allgemeinen Formel können auch in Form von Nasenpulvern oder Mitteln zur Einblasung verabfolgt werden.
Für diese Zwecke werden die Peptide in feinverteilter Form zusammen mit einem pharmakologisch verträglichen festen Trägerstoff, z.B. mit feinverteiltem Polyäthylenglykol (Carbowax® 1540), feinverteilter Lactose oder sehr fein verteiltem Siliciumdioxid (Cab-O-Sil), verabfolgt.
Derartige Mittel können auch andere Zusatzstoffe in feinverteilter Form, wie Konservierungsmittel, Puffer oder oberflächenaktive Mittel, enthalten.
Die Verbindungen der allgemeinen Formel können auch in Form von lange wirkenden Mitteln mit langsamer Wirkstoffabgabe oder Depotwirkung, wie nachstehend erläutert, vorzugsweise durch intramuskuläre Injektion oder durch Implantation, verabreicht werden.

Häufig ist es wünschenswert, die Verbindungen der allgemeinen Formel kontinuierlich über längere Zeiträume hinweg in Form von lange anhaltenden Präparaten mit langsamer Wirkstoffabgabe oder Depotwirkung zu verabreichen. Derartige Zubereitungsformen können entweder ein pharmakologisch verträgliches Salz der Wirkstoffverbindung mit einer geringen Löslichkeit in Körperflüsssigkeit, beispielsweise Salze mit Embonsäure, Gerbsäure oder Carboxymethylcellulose, oder die Verbindung der allgemeinen Formel in Form eines wasserlöslichen Salzes zusammen mit einem Schutzmittel, das eine rasche Freisetzung verhindert, enthalten. Im letztgenannten Fall kann beispielsweise die Verbindung der allgemeinen Formel mit nicht-antigener, teilweise hydrolysierter Gelatine in die Form einer viskosen Flüssigkeit gebracht oder an einem pharmakologisch verträglichen, festen Trägerstoff absorbiert werden. Der Wirkstoff kann auch in Form einer Suspension in einem pharmakologisch verträglichen, flüssigen Trägerstoff verabreicht werden. Ferner ist auch die Herstellung von Gelen oder Suspensionen mit einem nicht-antigenen Schutzhydrokolloid, z.B. Natriumcarboxymethylcellulose, Polyvinylpyrrolidon, Natriumalginat, Gelatine, Polygalacturonsäuren, oder bestimmten Mucopolysacchariden, zusammen mit wäßrigen oder nicht-wäßrigen, pharmakologisch verträglichen, flüssigen Trägerstoffen, Konservierungsmitteln oder oberflächenaktiven Mitteln möglich. Beispiele für derartige Präparate finden sich in üblichen pharmakologischen Handbüchern, wie Remintons's Pharmaceutical Sciences, Mack Publishing Co., Easton, Pa., 1975. Lang wirkende Präparate mit langsamer Wirkstoffabgabe erhält man auch durch Mikroverkapselung in einem pharmakologisch verträglichen Überzugsmaterial, wie Gelatine, Polyvinylalkohol oder Ethylcellulose. Bezüglich weiterer Beispiele für Überzugsmaterialien und Verfahren zur Mikroverkapselung wird auf J.A. Herbig in 〉〉Encyclopedia of Chemical Technology〈〈, Bd. 13,2. Aufl., Wiley, New York, 1967, S. 436 bis 456 verwiesen. Eine andere Möglichkeit besteht darin, einige der vorstehend aufgeführten festen Präparate, beispielsweise bestimmte wenig wasserlösliche Salze oder Dispersionen oder Adsorbate der Salze mit festen Trägerstoffen, beispielsweise Dispersionen in einem neutralen Hydrogel eines Polymerisats von Äthylenglykolmethacrylat oder ähnlichen vernetzten Monomeren; (vgl. US-PS 35 51 556) in Form von Pellets zuzubereiten, die die vorstehend angegebenen Wirkstoffmengen freisetzen. Diese Pellets können subkutan oder intramuskulär implantiert werden.
Bestimmte Verbindungen der allgemeinen Formel, die in Wasser wenig löslich sind, können als Suspensionen in einer wäßrigen Lösung oder einer Emulsionsgrundlage zubereitet werden. Suspensionen auf Wasserbasis werden mit Hilfe von Netzmitteln, wie Kondensationsprodukte von Polyäthylenoxid und Alkylphenolen, Fettalkoholen oder Fettsäuren und Suspendiermitteln, wie hydrophilen Kolloiden, z.B. Polyvinylpyrrolidon, hergestellt. Suspensionen auf Emulsionsbasis werden durch Suspendieren des Peptids mit Hilfe eines Netzmittels und Suspendiermitteln in der Emulsionsbasis hergestellt, wobei die vorstehend erläuterten Emulgiermittel verwendet werden. Suspensionen können zusätzlich Puffer und/oder Süßstoffe, Aromastoffe, Farbstoffe, Konservierungsmittel und Antioxidationsmittel enthalten.
Für die parenterale Verabfolgung wird die Verwendung eines wasserlöslichen Salzes der allgemeinen Formel in wäßriger, steriler Lösung bevorzugt. Beispiele für Konservierungsmittel sind p-Hydroxybenzoesäuremethyl- und - propylester, die zusammen mit anderen löslichen Bestandteilen, wie zur Herstellung von isotonen Lösungen ausreichenden Mengen an Natriumchlorid oder Glucose, zugesetzt werden. Peptide der allgemeinen Formel, die in Wasser wenig löslich sind, können auch intramuskulär in Form von Lösungen oder Suspensionen in sterilen, flüssigen, nicht-wäßrigen Trägern verabfolgt werden, beispielsweise in pflanzlichen oder tierischen Ölen. Dabei können gegebenenfalls die vorgenannten zusätzlichen Lösungsbestandteile oder Suspendiermittel enthalten sein.

Die Wirksamkeit der erfindungsgemäß definierten Verbindungen wurde auf ihre in vitro-Zytotoxizität geprüft.
Die Untersuchungen auf Inhibition der Kolonienbildung in Weichagar (colony assay) wurden in Anlehnung an die Methode von Hamburger und Salmon (A.W. Hamburger, S.E. Salmon. Primary bioassay of human myeloma stem cells. J. Clin. Invest. 60:846-854(1977), A.W. Hamburger and SE Salmon. Primary bioassay of human tumor stem cells. Science 197:461-463(1977) durchgeführt. Die Tumor-Zellen wurden in RPMI-1640-Medium mit 20 % fötalem Kälberserum und 0,3 % Agar in Anwesenheit verschiedener Konzentrationen der Testsubstanz bei 37°C, 95 % relativer Luftfeuchtigkeit und 5 % CO₂ inkubiert. Es wurden jeweils Dreifachwerte erhoben.
Die Inkubationszeit war 6 Tage für L1210 Mausleukämie und 8 Tage für KB-menschliches Schleimhaut-Karzinom.
Anschließend wurden die gebildeten Kolonien (> 50 Zellen) gezählt.
Die Konzentration der Testsubstanz, die eine Hemmung der Kolonienbildung von 90 % (EC₉₀) ergab, wurde graphisch bestimmt.

Die Ergebnisse der in vitro-Untersuchungen von einigen Verbindungen sind in der nachstehenden Tabelle gezeigt.
Die Verbindungen mit der höchsten Aktivität gegen KB-Zellen sind D-20 994, D-20 995 und D-22 267, gegen L 1210 Zellen D-21 979 und D-22 628.

| Substanz | Beschreibung | Zytotoxität in vitro EC₉₀ | |
|---|---|---|---|
| | | L 1210 µg/ml | KB µg/ml |
| D-20 989 | H-ORN(ORN)-NHC₁₈H₃₇.3HCL | 3.1 | 1.3 |
| D-20 990 | H-ORN[(ORN)ORN]-NHC₁₈H₃₇ 4HCl | > 10 | 1.7 |
| D-20 994 | H-LYS(LYS)-NHC₁₈H₃₇.3HBr | 3.2 | 0.8 |
| D-20 995 | H-LYS[(LYS)LYS]-NHC₁₈H₃₇.4HBr | > 3 | 0.9 |
| D-21 621 | H-LYS(LYS)-NHC₁₈H₃₇.3HCl | 2.2 | 1.4 |
| D-21 979 | H-THZ-NHC₁₄H₂₉ | 0.3 | 3.1 |
| D-21 980 | H-THZ-NHC₁₆H₃₃ | 3.4 | 3.1 |
| D-22 267 | H-LYS-LYS-NHC₁₈H₃₇ | 3.1 | 0.3 |
| D-22 628 | H-D-LYS(D-LYS)-NHC₁₈H₃₇.3HCL | 0.8 | 1.1 |

Die Kurzbezeichnungen der Verbindungen geben einen Hinweis auf die Verknüpfung der Di- und Tripeptidverbindungen. So bedeutet zum Beispiel H-LYS-LYS-NH-R, daß zwischen den Aminosäuren eine α-Verknüpfung vorliegt, während H-LYS(LYS)-NH-R eine endständige ω-Verknüpfung der Aminosäuren bedeutet. Bei den Aminosäure-, Dipeptid- und Tripeptidformeln ohne Chiralitätsangaben handelt es sich immer um die L-Formen, während die D-Formen gesondert als solche gekennzeichnet sind.

Die Verbindung H-LYS(LYS)-NHC₁₈H₃₇.3HCL (D-21621) wurde gesondert an humanen, in die Nacktmaus transplantierte Tumoren getestet. Am kleinzelligen Bronchialkarzinom LXFS 538 wurde mit der Dosis von 45 mg/kg, gegeben i.p. am Tag 1,5 und 9 eine Tumorhemmung auf 37 % der Kontrolle erzielt.
Am Ovarialkarzinom OVXF 899 erzielte die Verbindung unter den gleichen Bedingungen eine Tumorhemmung auf 46 % der Kontrolle. Dieser Tumor ist gegen sämtliche Standardzytostatika resistent.

Die Verbindungen der allgemeinen Formel werden nach bekannten Methoden synthetisiert, wie zum Beispiel durch die klassischen Lösungskupplungen. Man geht im allgemeinen in der Weise vor, daß das langkettige Alkylamin mit der geschützten Aminosäure umgesetzt wird und nachfolgend die Schutzgruppen in an sich bekannter Weise entfernt werden.
Klassische Lösungssynthese ist ausführlich in der Behandlung "Methoden der Organischen Chemie (Houben-Weyl).
Synthese von Peptiden", E. Wünsch (Herausgeber) (1974), Georg Thieme Verlag, Stuttgart, BRD beschrieben.

Unter den Klassen von α-Aminoschutzgruppen können beispielsweise Fluorenylmethoxycarbonyl (Fmoc) oder t-Butoxycarbonyl (BOC) genannt werden. Eine Schutzgruppe für die Hydroxylgruppe des Ser kann zum Beispiel Benzyl (Bzl) und 2,6-Dichlorbenzyl (DCB) sein. Beispielshaft für angebrachte Seitenkettenaminoschutzgruppen von Lys oder Orn sind Benzyloxycarbonyl (Z) und 2-Chlorbenzyloxycarbonyl [Z-(2-Cl)].

Für die stufenweise Kondensation von Aminosäuren eignen sich besonders gut aktivierte Ester von Benzyloxycarbonylaminosäuren, wie zum Beispiel N-Hydroxysuccinimidester oder 2,4,5-Trichlorphenylester und 4-Nitrophenylester. Die Aminolyse der beiden letzteren Aktivester läßt sich sehr gut durch N-Hydroxyverbindungen, die in etwa die Acidität der Essigsäure besitzen, wie zum Beispiel 1-Hydroxybenzotriazol katalysieren.
Als intermediäre Aminoschutzgruppen bieten sich abhydrierbare Gruppen wie zum Beispiel der Benzyloxycarbonylrest (= Z-Rest) oder schwach sauer abspaltbare Gruppen wie zum Beispiel der 2-(p-Diphenylisopropyloxycarbonyl- oder 2-(3,5-Dimethoxyphenyl)-isopropyloxy-carbonylrest an.
Die stufenweise Kondensation erfolgt durch Synthese aus den entsprechenden, gegebenenfalls in üblicher Weise geschützten Aminosäuren, in herkömmlicher Weise.

Die Darstellung von

H-Orn-NHC₁₆H₃₃.2HBr

H-Orn-NHC₁₈H₃₇.2HBr

H-Orn-Orn-NHC₁₈H₃₇.3HCl

H-Orn(Orn)-NHC₁₈H₃₇.3HCl

ist von G.Kellner und M. Liefländer in Z.Naturforsch. B, Chem. Sci, 46 (8) 1098-1104 (1991) beschrieben.
Entsprechend der darin angeführten Vorschrift können ebenfalls die neuen Verbindungen hergestellt werden. Für die Derivate H-LYS[(LYS)LYS]-NHC₁₈H₃₇.4HBr und H-D-LYS(D-LYS)-NHC₁₈H₃₇.3HCl wird beispielshaft die Herstellungsvorschrift angegeben

Z-Lys(Z-Lys(Boc))-OH

Eingesetzte Mengen:

| | |
|---|---|
| Z-Lys(Boc)-OSu | 7,16 g (15,0 mmol) |
| Z-Lys-OH | 4,63 g (16,5 mmol) |
| Triethylamin | 2,5 ml (18,0 mmol) |

Durchführung: Der Succinimidester wird in 50 ml Dioxan gelöst. Dazu wird die in 50 ml gelöste Aminkomponente mit der Base bei Raumtemperatur gegeben. Nach 20 h wird das Reaktionsgemisch gemäß folgender allgemeinen Vorschrift aufgearbeitet.

Je 1 mmol des N-geschützten Aminosäure- bzw. Peptidsuccinimidester wird je nach Löslichkeit in 3 - 6 ml Dioxan gelöst. Bei Raumtemperatur wird unter kräftigem Rühren eine Mischung aus je 1,2 mmol Triethylamin und 1,1 mmol Aminokomponente in 3 - 6 ml Wasser (die Wassermenge sollte dem Dioxanvolumen entsprechen) auf einmal zugegeben.
Nach 24 h Reaktionszeit wird mit 0,5 M Citronensäurelösung angesäuert, je mmol Succinimidester 2,5 ml Säure und sofort mehrmals mit Ethylacetat(EE) extrahiert. Die hierfür benötigte Menge an EE steigt mit der Peptidlänge erheblich an. Die vereinigten organischen Phasen werden dreimal mit Wasser gewaschen und über Na₂SO₄ getrocknet. Oftmals ist es nicht zweckmäßig, das Lösungsmittel vollständig abzuziehen, da das ölige Produkt dann auch mit Hilfe von Fällungsmitteln nicht zur Kristallisation gebracht werden kann. Als Fällungsmittel dienen in der Regel Disopropylether, Hexan, Diethylether oder Petrolether. In Einzelfällen ist eine Fällung als DCHA-Salz günstiger.

Meist fallen schon beim Abziehen des EE erste Kristalle an; die Kristallisation wird bei 4°C unter Zusatz von wenig Diisopropylether vervollständigt.
- Rohausbeute:: 9,45 g (14,7 mmol, 98 %).
Schmp.: 138-139°C
Umkristallisation aus EE
- Reinausbeute:: 9,0 g (14 mmol, 93 %)
Schmp.: 140-141°C

Z-Lys(Z-Lys(Boc))-OSu

Eingesetzte Mengen:

| | |
|---|---|
| Z-Lys(Z-Lys(Boc))-OH | 7,71 g (12,0 mmol) |
| N-Hydroxysuccinimidester | 1,38 g (12,0 mmol) |
| DCC | 2,46 g (12,0 mmol) |

Durchführung: Gemäß der folgenden allgemeinen Vorschrift
Je 1 mmol N-geschütztes Aminosäure- bzw. Peptidderivat und 1 mmol N-Hydroxysuccinimid werden in je 5 ml THF gelöst und auf -10°C gekühlt. Danach setzt man je 1 mmol DCC zu, und rührt 1,5 h bei - 10°C und 24 h bei Raumtemperatur. Der ausgefallene, fein kristalline N,N'-Dicyclohexylharnstoff wird abfiltriert und mit EE gewaschen. Das Filtrat wird auf wenige ml eingeengt und das anfallende Öl entweder durch Verreiben mit Petrolether oder durch Fällung aus EE/Diisopropylether zur Kristallisation gebracht. Die Umkristallisation erfolgt aus geeigneten Lösungsmitteln.
Bei Ansätzen mit Eduktmengen über 12 mmol hinaus können sich verstärkt sehr schwer zu entfernende Nebenprodukte bilden.

Es fällt die hervorragende Löslichkeit des Peptids in THF auf.
- Rohausbeute:: 8,6 g (11,6 mmol, 97 %)
Schmp.: 127-129°C
Umkristallisation aus Ethanol
- Reinausbeute:: 8,1 g (10,9 mmol, 91 %)
Schmp.: 130-132°C

Boc-Lys(Z-Lys(Z-Lys(Boc)))-OH

Eingesetzte Mengen:

| | |
|---|---|
| Z-Lys(Z-Lys(Boc))-OSu | 7,40 g (10,0 mmol) |
| Boc-Lys-OH | 2,71 g (11,0 mmol) |
| Triethylamin | 1,70 ml (12,0 mmol) |

Durchführung: Der Succinimidester wird in 50 ml Dioxan gelöst. Bei Raumtemperatur werden die in 50 ml H₂O gelöste Aminkomponente und NEt₃ zugegeben und 24 h gerührt. Nach dem Ansäuern mit Citronensäure und dreimaligen Extrahieren mit EE werden die vereinigten organischen Phasen mehrmals mit H₂O gewaschen und über Na₂SO₄ getrocknet. Die Lösung wird auf wenige ml eingeengt. Dabei bilden sich die ersten Kristalle am Kolbenrand. Diese werden unter Umschütteln in den Rückstand gebracht, worauf sehr schnell die Kristallisation einsetzt. Um ein Verklumpen zu verhindern, wird mehrmals EE zugegeben. Die Kristallisation wird bei 4°C vervollständigt.
- Rohausbeute:: 8,54 g (9,8 mmol, 98 %)
Schmp.: 153 - 154°C
Umkristallisation erfolgt aus Methanol/EE/Diisopropylether
- Reinausbeute:: 8,19 g (9,4 mmol, 94 %)
Schmp.: 154-155°C

Boc-Lys(Z-Lys(Z-Lys(Boc)))-OSu

Eingesetzte Mengen

| | |
|---|---|
| Boc-Lys(Z-Lys(Z-Lys(Boc)))-OH | 6,97 g (8,0 mmol) |
| N-Hydroxysuccinimid | 0,92 g (8,0 mmol) |
| DCC | 1,64 g (8,0 mmol) |

Durchführung: Das Peptid löst sich sehr schlecht in THF, so daß 80 ml THF und Beschallung im Ultraschallbad für den Lösevorgang nötig sind. Es wird auf -10°C gekühlt und das in 10 ml THF gelöste SuOH und das in 30 ml THF gelöste DCC getrennt zugegeben. Nach 1 h Rühren bei -10°C und 20 h bei Raumtemperatur ist das Reaktionsgemisch stark eingedickt. Der Brei wird in Methanol aufgenommen und der teilweise nicht gelöste Dicyclohexylharnstoff abfiltriert. Beim Einengen des Gemisches muß noch dreimal ausgefallener Harnstoff entfernt werden. Durch Zugabe von Diisopropylether erreicht man die Kristallisation des Produkts.
- Rohausbeute:: 7,74 g (8,0 mmol, 100 %)
Schmp.: 119-120°C
Umkristallisation erfolgt aus Ethanol
- Reinausbeute:: 7,17 g (7,4 mmol, 93 %)
Schmp.: 120-121°C

Boc-Lys(Z-Lys(Z-Lys(Boc)))-NHC₁₈H₃₇

Eingesetzte Mengen:

| | |
|---|---|
| Boc-Lys(L-Lys(Z-Lys(Boc)))-OSu | 4,84 g (5,0 mmol) |
| Octadecylamin | 1,35 g (5,0 mmol) |

Durchführung: Der Succinimidester wird in 50 ml Dioxan gelöst und zu einer Lösung des Amins in 30 ml CHCl₃ gegeben. Der Ansatz geliert nach 11 h. Durch Zugabe von CHCl₃ und Erwärmen auf 40°C erhält man wieder eine Lösung. Das Lösungsmittel wird entfernt und der Rückstand mit Diisopropylether zur Kristallisation gebracht.
- Reinausbeute:: 4,61 g (4,1 mmol, 82 %)
Schmp.: 139-142°C

H-Lys(H-Lys(H-Lys))-NHC₁₈H₃₇. 4HBr

Eingesetzte Mengen:

| | |
|---|---|
| Boc-Lys(Z-Lys(Z-Lys(Boc)))-NHC₁₈H₃₇. | 2,24 g (2,0 mmol) |
| HBr/Eisessig | 15,8 ml |

Die Verbindung wird mit kalter 33%iger HBr/Eisessig bei 4°C übergossen und 2-3 h bei 10°C unter Feuchtigkeitsausschluß gerührt. Das Hydrobromid fällt meistens schon während der Reaktion fast vollständig aus. Nach Zugabe von Diethylether (DE) wird das Produkt abgesaugt und gut mit DE gewaschen.
Das Rohprodukt riecht noch stark nach Benzylbromid und wird sofort mehrere Male umkristallisiert. Ist das Produkt im Essigsäure-Diethylether-Gemisch löslich, wird das Lösungsmittel am Wasserstrahlvakuum abgezogen und der Rückstand in wenig Ethanol aufgenommen. Durch Zugabe von DE oder EE kann das Produkt gefällt werden. Es wird durch Umkristallisation aus Methanol/EE gereinigt.
- Reinausbeute:: 1,52 g (1,6 mmol, 78 %)
Schmp.: sintert ab 226°C

H-D-Lys(D-Lys)-NH-C₁₈H₃₇3HCl

Boc-D-Lys-OH wird im äquimolaren Mengen Triethylamin und Boc-D-Lys(Boc)-OSu in DMF zum geschützten Dipeptid Boc-D-Lys(Boc-D-Lys(Boc))-OH umgesetzt. Dieses wird mit DPPA und einem 10proz. Überschuß Octadecylamin in THF zum Boc-D-Lys(Boc-D-Lys(Boc))-NH-C₁₈H₃₇ gekuppelt. Das über Kieselgel säulenchromatographisch gereinigte Produkt wird aus THF/Diisopropylether auskristallisiert. Fp.: 70-71°C. Nach Abspaltung der Schutzgruppen mit TFA wird das Trihydrochlorid des Dipeptidamids mit HCl in Ether aus Ethanol ausgefällt und dann aus Ethanol umkristallisiert.
Fp.: 215°C (Zers.)

## Patentansprüche

1. Langkettige unverzweigte Alkylamide der allgemeinen Formel
CH₃-(CH₂)ₙ-NH-R,
wobei n = 11 - 20 und R = Aminosäure, deren D- und L- Enantiomeren, Di- und Tripeptide, durch N-Schutzgruppen derivatisierte Formen und endständige Ethylester sowie deren physiologisch verträgliche Salzformen als pharmazeutische Wirkstoffe mit antiproliferativen und entzündungshemmenden Eigenschaften.

2. Langkettige Alkylamide nach Anspruch 1, wobei n = 15 - 19 und die Aminosäurebausteine R = Lysin, Serin, Prolin, Thiaprolin, Hydroxyprolin und Ornithin sind.

3. Langkettige Alkylamide nach Anspruch 1, wobei n = 17.

4. Langkettige Alkylamide nach Anspruch 1 zur Anwendung als antiproliferative Wirkstoffe.

5. Langkettige Alkylamide nach Anspruch 1, wobei die N-Schutzgruppen Z = Benzyloxycarbonyl, BOC = tert. butoxycarbonyl und die Salzformen Hydrochloride und Hydrobromide bedeuten.

6. Arzneimittel enthaltende antiproliferative und entzündungshemmende Wirkstoffe nach Anspruch 1, und pharmazeutische Hilfs- und Trägerstoffe.

7. Verwendung eine Verbindung nach Anspruch 1 als Wirkstoff und gegebenenfalls pharmazeutisch übliche Verdünnungs-, Träger-, Füll- oder Hilfsstoffe zur Behandlung von proliferativen und phlogistischen Krankheitszuständen.

8. Verwendung einer Verbindung nach Anspruch 1 zur Herstellung eines Arzneimittels zur Behandlung von proliferativen und phlogistischen Krankheitszuständen.

9. H-Lys-Lys-NH-(CH₂)₁₇-CH₃.3HX, wobei X = Cl, Br.

10. H-Lys(Lys)-NH-(CH₂)₁₇-CH₃.3HX, wobei X = Cl, Br

11. H-D-Lys(D-Lys)-NH-(CH₂)₁₇-CH₃.3 HX, wobei X = Cl, Br

12. H-THZ-NH-(CH₂)₁₃-CH₃.HCl
